(19) **Europäisches Patentamt / European Patent Office / Office européen des brevets**

(11) **EP 3 673 077 B1**

(12) **EUROPEAN PATENT SPECIFICATION**

(45) Date of publication and mention
of the grant of the patent:
**08.04.2026   Bulletin 2026/15**

(21) Application number: **18759306.6**

(22) Date of filing: **23.08.2018**

(51) International Patent Classification (IPC):
**C12Q 1/66** *(2006.01)*      **G01N 33/542** *(2006.01)*

(52) Cooperative Patent Classification (CPC):
**G01N 33/542; C12Q 1/66**

(86) International application number:
**PCT/EP2018/072758**

(87) International publication number:
**WO 2019/038375 (28.02.2019 Gazette 2019/09)**

(54) **DETECTION DEVICE FOR BIOLUMINESCENT DETECTION OF BIOMARKERS FROM A BIOLOGICAL FLUID SAMPLE USING LUMINESCENT SENSING PROTEINS**

DETEKTIONSVORRICHTUNG ZUR BIOLUMINESZENZ-DETEKTION VON BIOMARKERN AUS EINER BIOLOGISCHEN FLÜSSIGPROBE MIT LUMINESZENZ-SENSITIVEN PROTEINEN

DISPOSITIF DE DÉTECTION POUR LA DÉTECTION BIOLUMINESCENTE DE BIOMARQUEURS À PARTIR D'UN ÉCHANTILLON DE FLUIDE BIOLOGIQUE À L'AIDE DE PROTÉINES DE DÉTECTION LUMINESCENTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.08.2017   US 201762550001 P**

(43) Date of publication of application:
**01.07.2020   Bulletin 2020/27**

(73) Proprietor: **Technische Universiteit Eindhoven 5612 AZ Eindhoven (NL)**

(72) Inventors:
• **TENDA, Keisuke**
  **Tokyo 179-0083 (JP)**
• **ARTS, Remco**
  **5685 GC Best (NL)**
• **CITTERIO, Daniel**
  **Kawasaki 212-0055 (JP)**
• **TOMIMURO, Kosuke**
  **Yokohama 223-0061 (JP)**
• **MERKX, Maarten**
  **5685 AG Best (NL)**

(74) Representative: **Algemeen Octrooi- en Merkenbureau B.V.**
**P.O. Box 645**
**5600 AP Eindhoven (NL)**

(56) References cited:
**EP-A2- 1 925 320**      **WO-A1-2013/155553**
**WO-A1-2015/007317**      **WO-A1-2015/067302**
**US-B1- 6 395 504**

• **RUDOLF GRISS ET AL: "Bioluminescent sensor proteins for point-of-care therapeutic drug monitoring", NATURE CHEMICAL BIOLOGY, vol. 10, no. 7, 8 June 2014 (2014-06-08), pages 598 - 603, XP055146028, ISSN: 1552-4450, DOI: 10.1038/nchembio.1554**
• **N. R. POLLOCK ET AL: "A Paper-Based Multiplexed Transaminase Test for Low-Cost, Point-of-Care Liver Function Testing", SCIENCE TRANSLATIONAL MEDICINE, vol. 4, no. 152, 19 September 2012 (2012-09-19), US, pages 152ra129 - 1, XP055527892, ISSN: 1946-6234, DOI: 10.1126/scitranslmed.3003981**

- ANONYMOUS: "Nano-Glo Luciferase Assay System", 1 July 2015 (2015-07-01), pages 1 - 17, XP055527903, Retrieved from the Internet <URL:https://www.promega.com/-/media/files/resources/protocols/technical-manuals/101/nanoglo-luciferase-assay-system-protocol.pdf> [retrieved on 20181128]
- REMCO ARTS ET AL: "Detection of Antibodies in Blood Plasma Using Bioluminescent Sensor Proteins and a Smartphone", ANALYTICAL CHEMISTRY, vol. 88, no. 8, 7 April 2016 (2016-04-07), US, pages 4525 - 4532, XP055527905, ISSN: 0003-2700, DOI: 10.1021/acs.analchem.6b00534
- RODA ALDO ET AL: "Progress in chemical luminescence-based biosensors: A critical review", BIOSENSORS AND BIOELECTRONICS, ELSEVIER SCIENCE LTD. UK, AMSTERDAM, NL, vol. 76, 19 June 2015 (2015-06-19), pages 164 - 179, XP029300766, ISSN: 0956-5663, DOI: 10.1016/J.BIOS.2015.06.017
- LIN XUE ET AL: "Bioluminescent Antibodies for Point-of-Care Diagnostics", ANGEWANDTE CHEMIE INTERNATIONAL EDITION, vol. 56, no. 25, 16 May 2017 (2017-05-16), DE, pages 7112 - 7116, XP055527910, ISSN: 1433-7851, DOI: 10.1002/anie.201702403

## Description

### FIELD OF THE INVENTION

This invention relates to devices for the detection and quantification of analytical markers present in a fluid sample.

### BACKGROUND OF THE INVENTION

Microfluidic paper-based analytical devices (μPADs) represent a class of microfluidic devices characterized by (i) low material costs, (ii) external power-free sample transport driven by capillary forces and (iii) being lightweight, disposable and deliverable to end users. Research has come a significant way in converting labor-intensive and complicated clinical assays into more user-friendly formats on paper platforms. Nevertheless, several hurdles remain that hamper the more widespread point-of-care (POC) application of μPADs. Quantitative colorimetric assays for example, require measures to eliminate the influence of environmental light conditions, whereas fluorescence-based detection potentially suffers from paper autofluorescence or light scattering, and additionally requires the use of an excitation light source. Liquid handling steps pose another challenge to assay simplification.

The widespread ELISA and LUMINEX methods require multiple liquid handling steps (pipetting, incubation, washing, signal generation, etc.), which pose significant challenges in translating those assays from dedicated laboratory instruments into simple paper-based microfluidic systems. While lateral flow immunochromatographic assays do not suffer from these drawbacks, they have limited sensitivity and generally show poor quantitative performance. This applies for example to assays for the detection of antibodies in disease diagnostics and in drug monitoring-guided dose optimization of therapeutic antibodies.

The publication of Griss and coworkers (Nat. Chem. Biol. 2014. 10:598-603) and the related patent WO 2015/007317 describe paper-based biosensors using bioluminescence detection.

The present invention addresses some of these problems and hurdles and provides new technology and methodology towards widespread point-of-care (POC) application of μPADs.

### SUMMARY OF THE INVENTION

The present invention provides a detection device for bioluminescent detection of analytical targets from a biological or a non-biological fluid sample using luminescent sensing proteins. In detail, the invention relates to a detection device for bioluminescent detection of analytical targets from a fluid sample using luminescent sensing proteins, comprising: (a) a first lamination layer with an opening for receiving the fluid sample; (b) a separation membrane for separating part of the fluid sample received through the opening of the first lamination layer; (c) a plastic film layer with sewn-in cotton thread, wherein the thread comprises two intertwisted threads, one impregnated with luminescent sensing proteins and the other impregnated with luciferin, thereby spatially separating the luminescent sensing proteins and luciferin to prevent their premature mixing, wherein, upon receiving the fluid sample, the luciferin and luminescent sensing proteins generate a bioluminescent signal for the detection of the analytical targets; and (d) a second lamination layer, which together with the first lamination layer encases and immobilizes the separation layer, and the plastic film layer with sewn-in cotton thread into a single vertically stacked assembly detection device.

Examples of biological fluid samples are blood, blood serum, blood plasma, urine, tear fluid, or saliva. Examples of non-biological fluid samples are drinking, environmental and waste water samples, or food or soil extracts. Examples of analytical targets are biomarkers, antibodies, antigens, proteins, drugs, or nucleic acids.

In general, the single vertically stacked assembly detection device enables a pump-free transport mechanism of the fluid sample by means of capillary forces, gravity or a combination thereof. A specific example of the detection device, described herein, is where the separation membrane is a blood cell filtration membrane.

The first lamination layer, which is the top layer in the stack, has an opening for receiving the fluid sample. Underneath the first lamination layer is the separation membrane which separates part of the fluid sample received through the opening of the first lamination layer.

As described herein, underneath the separation membrane is the first porous layer which is impregnated with a luciferin. The first porous layer receives the separated fluid sample. At the first porous layer the luciferin dissolves into the fluid sample.

As described herein, underneath the first porous layer is the second porous layer which is patterned with detection areas separated by hydrophobic barriers. The detection areas are impregnated with one or more different luminescent sensing proteins for the detection of the analytical targets.

The second porous layer described herein receives the luciferin dissolved fluid sample, as it is passed through the first porous layer, which then interacts with luminescent sensing proteins. The case for the bioluminescence is the oxidation of the luciferin substrate (furimazine) by the luciferase present in the luminescent sensing protein (NanoLuc in the antibody sensor protein LUMABS). The detection device will also emit bioluminescence in the absence of an analytical target. The analytical target modulates the color (i.e. wavelength) of the emitted bioluminescence. Luciferin can comprise furimazine, coelenterazine or derivatives thereof. The luciferase integrated into e.g. a LUMABS can be "NanoLuc", Renilla luciferase (RLuc), firefly luciferase (FLuc), etc.. In other words, the invention would also include bioluminescent sensing proteins not using the NanoLuc enzyme.

The bottom layer of the stack is the second lamination layer, which together with the first lamination layer encases and immobilizes the separation layer, and the plastic film layer with sewn-in cotton thread into the single vertically stacked assembly detection device.

In an aspect described herein, the first and second porous layer could be one single layer, whereby the aspects of the two layers are integrated into the single layer.

In another aspect described herein, the first and second porous layer could be one single layer, whereby the aspects of the two layers are integrated into the single layer by the use of impregnated threads.

The first porous, the second porous layer and/or the single porous layer could be made out of paper, cellulose fibers, glass fibers, threads, bamboo, cloth, or any combination thereof.

Another embodiment of the detection device is to further have a reference scale or system for the (measurement or quantification of) bioluminescent emission signals.

An aspect described herein is an integrated paper-based analytical system relying on ratiometric bioluminescence detection in a single drop of whole blood that allows quantitative detection of biomarkers in a highly user-friendly "just add the sample" manner. Bioluminescent ratiometric sensing is particularly useful for colorimetric assays on paper platforms, as it eliminates important challenges associated with colorimetric detection on μPADs related to the use of external light sources, environmental light conditions and intensity-based signaling. The device design enables sample volume independent and fully reagent-free operation, including on-device blood plasma separation. User operation is limited to the application of a single drop (10 - 30 μL) of sample (serum, whole blood) and the acquisition of a photograph approximately 20 minutes after sample introduction, with no requirement for precise pipetting, liquid handling or analytical equipment except for a digital camera. Although a standard digital camera could be used in this invention for signal acquisition, other embodiments or applications of the 3D-μPAD devices may use a smartphone camera, allowing integration of signal detection and application-based analysis without a dedicated measurement instrument.

## BRIEF DESCRIPTION OF THE DRAWINGS

FIG. 1A — shows according to an exemplary embodiment of the invention a schematic representation of the LUMABS working principle with the green light emitting "closed form" and the blue light emitting "open form" protein sensor in the absence and presence of target antibody, respectively (NLuc: NanoLuc luciferase; mNG: mNeonGreen fluorescent protein).

FIG. 1B — shows according to an aspect described herein a schematic representation of a multi-layer 3D-μPAD. The plasma separation membrane (PSM) is placed on top, followed by a furimazine impregnated paper layer (light emitting substrate for NanoLuc enzyme, chemical structure shown in the inset) and a LUMABS impregnated patterned paper layer. All layers are kept together through lamination. Further information on the modification of each paper layer is available in FIGs. 5A-C.

FIG. 1C — shows according to an aspect described herein a schematic outline of the use of a 3D-μPAD for simultaneous detection of three different antibodies.

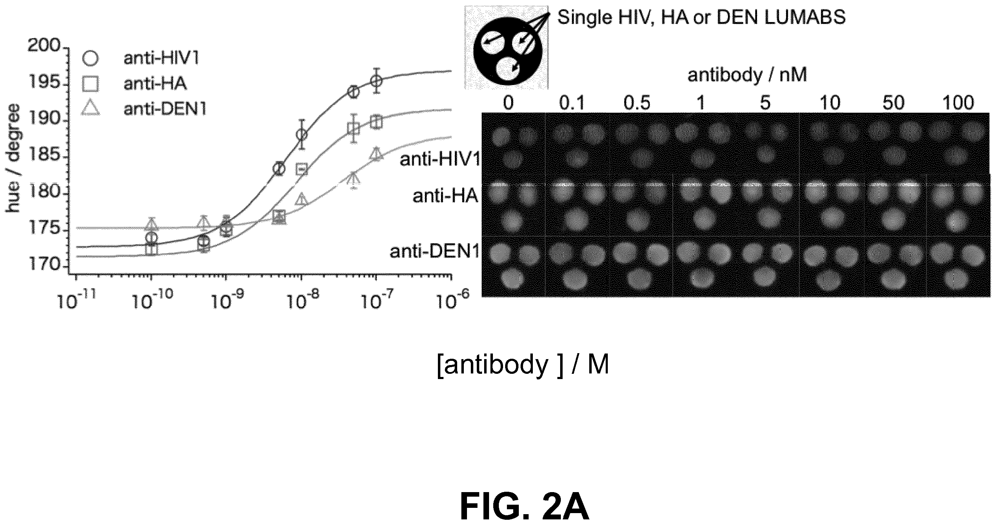

FIG. 2A — shows according to an aspect described herein typical colorimetric response curves and photographs of actually observed bioluminescence emission signals obtained with multi-layer 3D-μPADs after application of 20 μL aqueous antibody samples for devices with signal detection areas modified with in **FIG. 2A** identical amounts of one of three different LUMABS (100 fmol for anti-HIV-LUMABS and anti-DEN-LUMABS, 250 fmol for anti-HA- LUMABS), each targeting a different antibody (anti-HIV1, anti-HA, anti-DEN1). Error bars indicate the standard deviations for triplicate experiments using readouts from 3 different signal detection areas on one single device. Data recorded 15 min after sample application.

FIG. 2B — shows according to an aspect described herein typical colorimetric response curves and photographs of actually observed bioluminescence emission signals obtained with in **FIG. 2B** multi-layer 3D-μPADs after application of 20 μL aqueous antibody samples for devices with signal detection areas modified with 3 different amounts of anti-HIV-LUMABS. Error bars indicate the standard deviations for triplicate experiments using readouts from 3 separate devices per data point. Data recorded 15 min after sample application.

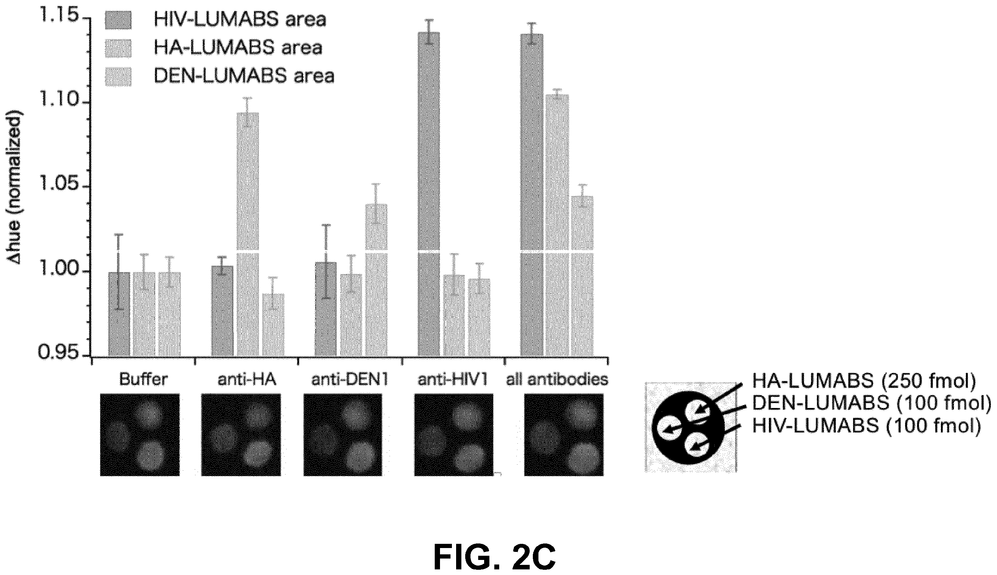

FIG. 2C — shows according to an aspect described herein typical colorimetric response curves and photographs of actually observed bioluminescence emission signals obtained with multi-layer 3D-μPADs after application of 20 μL aqueous antibody samples for devices with signal detection areas modified with in **FIG. 2C** colorimetric response (normalized Δhue) of 3D-μPADs responsive to multiple antibodies upon exposure to 33 nM of single antibodies or mixtures thereof. Error bars indicate the standard deviations for triplicate experiments using readouts from 3 separate devices per data point. Data recorded 15 min after sample application.

**FIG. 3A**     shows according to an aspect described herein a schematic representation of the sample volume independent fully laminated 3D-μPAD system.

**FIG. 3B**     shows according to an exemplary aspect described herein experimentally obtained hue values after application of various volumes of aqueous anti-HIV1 solutions (1 nM or 10 nM) to devices modified with anti-HIV-LUMABS; error bars indicate the standard deviations for triplicate readouts from 3 signal detection areas on one single device; data recorded 15 min after sample application.

**FIG. 3C**     shows according to an aspect described herein a schematic outline of the detection areas and photos showing the actual colorimetric response as recorded by the camera.

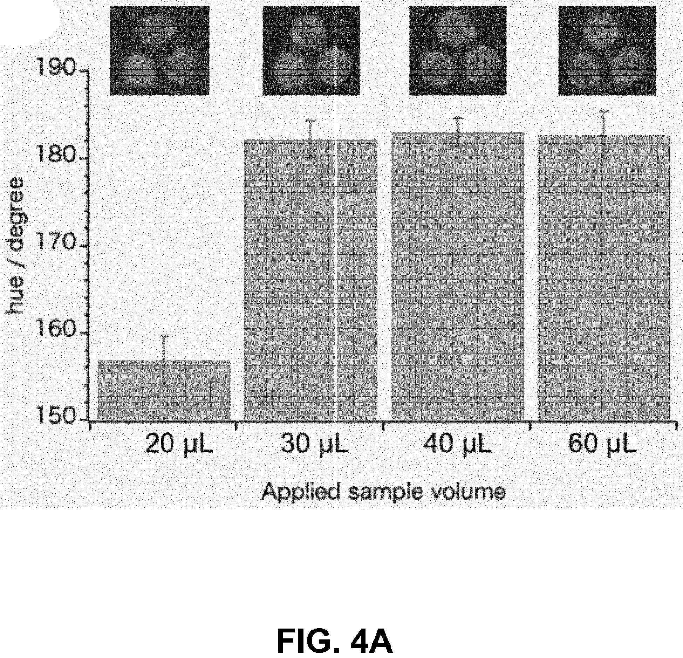

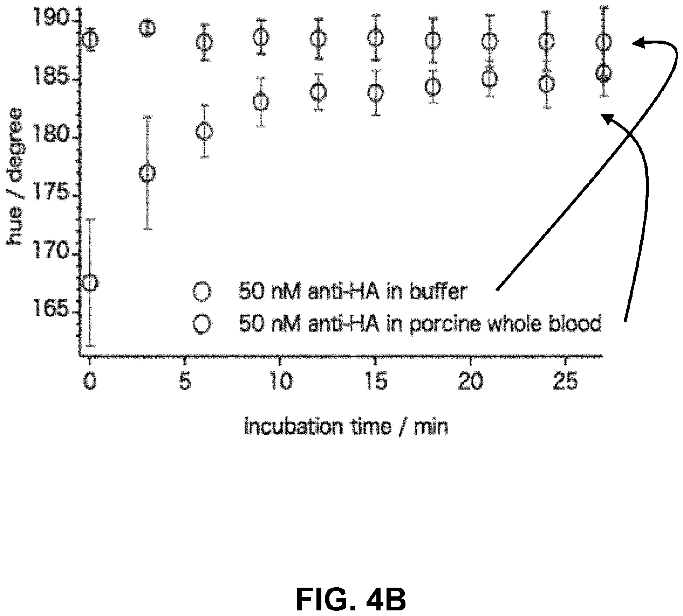

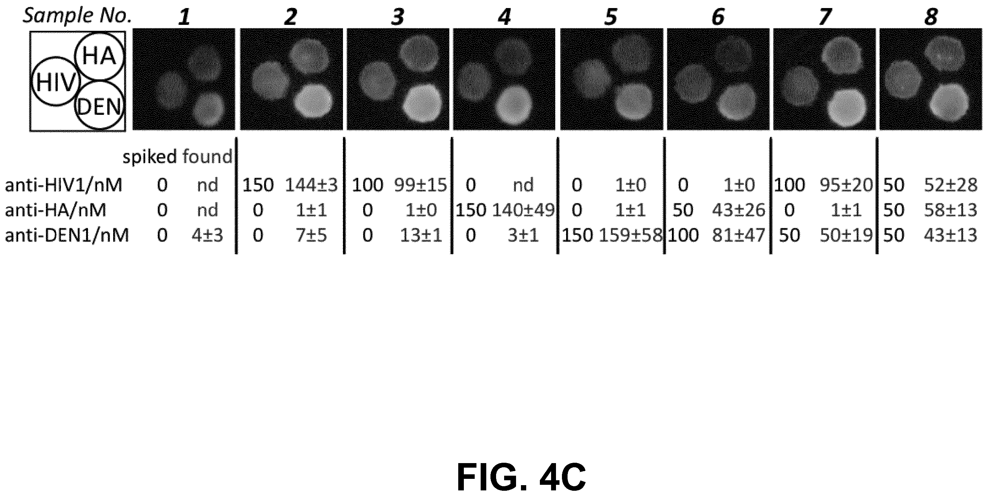

**FIGs. 4A-C**     show according to an aspect described herein 3D-μPADS applied to antibody assays in whole blood samples. **FIG. 4A** shows hue values obtained from variable amounts of porcine whole blood spiked with 50 nM anti-HA applied to an anti-HA targeting 3D-μPAD; data recorded 21 min after sample application; photos above the graph show the actual colorimetric response as recorded by the camera. **FIG. 4B** shows time-dependent bioluminescence emission hue values recorded from anti-HA-LUMABS modified 3D-μPADs after application of 20 μL aqueous anti-HA samples or 30 μL of anti-HA spiked porcine whole blood. In **FIGs. 4A-B** error bars indicate the standard deviations for readouts from 3 different signal detection areas on one single device. **FIG. 4C** shows images of signal detection areas of 3D-μPADs responsive to multiple antibodies recorded 21 min after application of antibody-spiked porcine whole blood samples; spiked and found antibody concentrations are indicated in black and red numbers, respectively; confidence intervals represent standard deviations for triplicate readouts from 3 separate devices.

**FIG. 5A**     shows according to an aspect described herein modification of the upper paper layer (furimazine impregnated) for 3D-μPADs.

**FIG. 5B**     shows according to an exemplary embodiment of the invention modification of the lower paper layer (LUMABS impregnated) for 3D-μPADs.

**FIG. 5C**     shows according to an aspect described herein the outline of wax pattern set in Adobe Illustrator CC; multiple detection areas impregnated with identical amounts of identical LUMABS (d-1), variable amounts of identical LUMABS (d-2), or different LUMABS (d-3).

**FIG. 6**     shows according to an exemplary embodiment of the invention multi-layer μTAD design and actual images.

**FIG. 7**     shows according to an exemplary embodiment of the invention the μTAD fabrication. LUMABS chosen from HIV-LUMABS, HA-LUMABS, and DEN-LUMABS. In cases of devices for assaying multiple antibodies with different LUMABS, a separate section of the thread can be used for each type of LUMABS.

**FIG. 8A**     shows according to an exemplary embodiment of the invention of a signal readout with the image showing light emission from 6 sensing areas on a single device. HUE values calculated from RGB values are used for data processing.

**FIG. 8B**     shows according to an exemplary embodiment of the invention of a signal readout with the regions of interest chosen automatically based on a defined cut off gray value.

## DETAILED DESCRIPTION

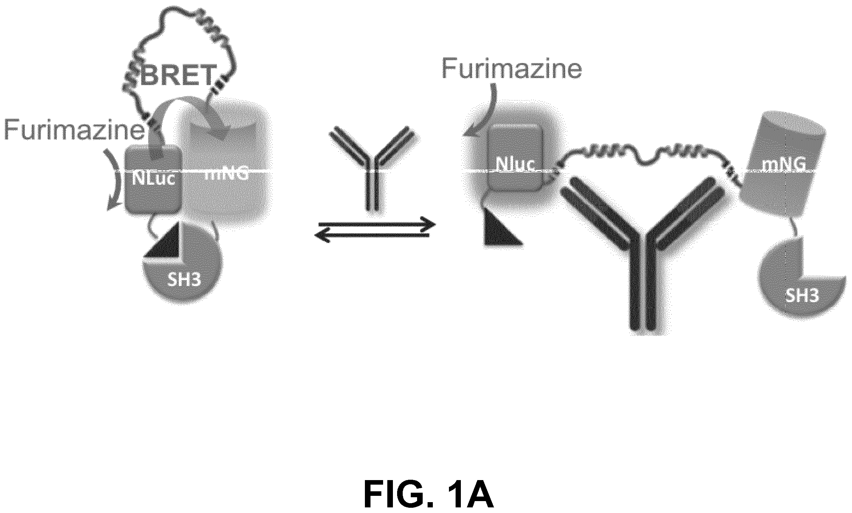

Some of the drawbacks of classical heterogeneous immunoassays were successfully addressed by introducing a new type of bioluminescence resonance energy transfer (BRET)-based immunoassay that integrates antibody binding and signal generation in a single protein switch referred to as LUMABS (**FIG. 1A**), making any washing steps unnecessary.

Antibody binding in LUMABS results in a readily detected change in emitted bioluminescence from green to blue. This ratiometric response offers significant advantages over intensity-based sensing approaches, which are inherently influenced by factors not related to target analyte concentration. These properties and the absence of background fluorescence and scattered excitation light allowed direct detection of antibodies in blood plasma with LUMABS using a mobile phone camera as detector. BRET-based switches are not limited to antibody detection as they can also be useful for therapeutic drug monitoring of low molecular weight compounds and nucleic acids.

BRET-based ratiometric sensing is particularly useful for colorimetric assays on paper platforms, since it eliminates important challenges for μPADs associated with external light sources, environmental light conditions and intensity-based signaling. An additional benefit of paper-based BRET signal detection compared to solution phase assays

**EP 3 673 077 B1**

is the suppressed absorption of the bioluminescent signal by blood components because of the short optical path length of thin paper layers. While the latter advantage has already been demonstrated by performing the final read-out on filter paper, current assay procedures still require complex and quantitative liquid handling steps such as cell separation, sample dilution, and substrate addition and mixing. These steps, while done in a laboratory setting, represent significant hurdles for the practical application of BRET-based diagnostics in user-friendly point-of-care-testing (POCT) by untrained users.

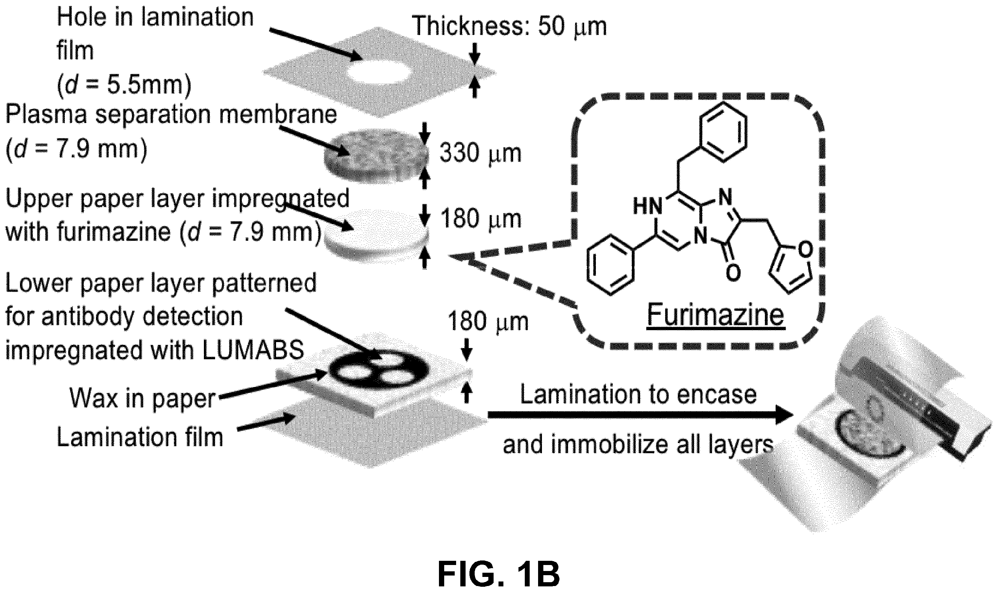

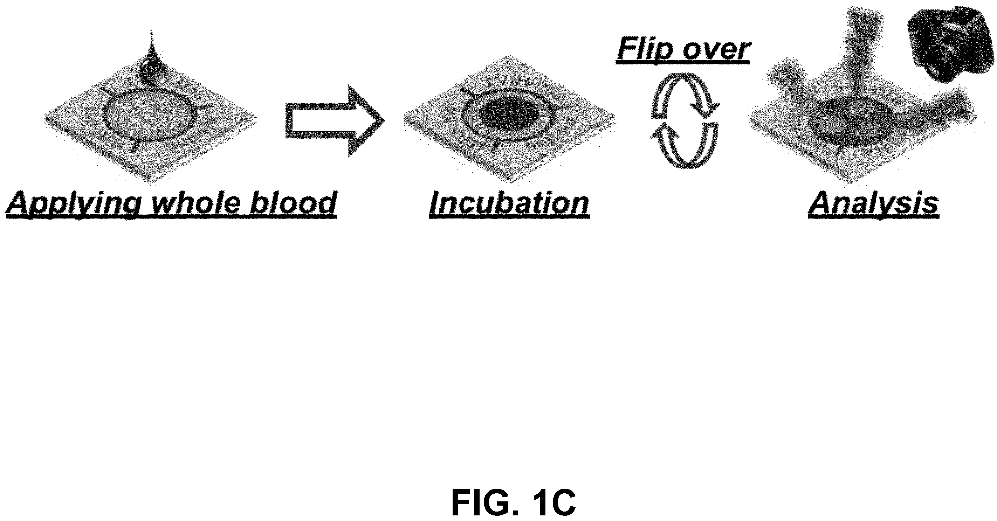

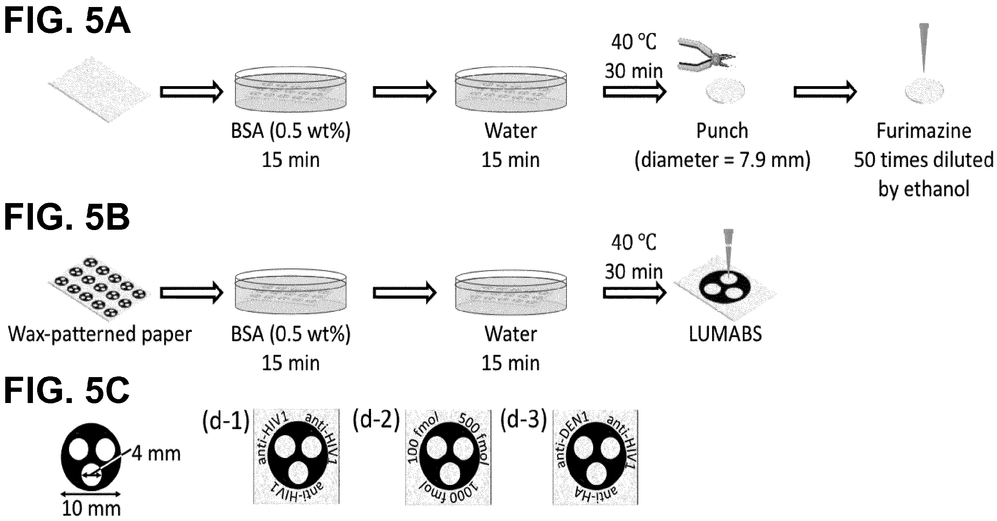

Described herein are fully integrated microfluidic paper-based analytical devices (μPAD) for use with bioluminescent BRET sensors, which are used for LUMABS-based detection of (multiple) antibodies in spiked whole blood samples. **FIG. 1B** shows the design of the device, which has multiple paper layers that are vertically arranged in a laminated 3D-μPAD design. The first layer (plasma separation membrane) serves as the sample pad, where cellular components are separated from a whole blood sample. The second layer contains the substrate (furimazine), which is dissolved into the vertically flowing sample liquid and carried along to the third layer containing the BRET switching protein (LUMABS). The arrangement of paper layers for furimazine (non-patterned upper paper layer) and LUMABS immobilization (patterned lower paper layer) was chosen for simplicity in device fabrication, since it eliminates the requirement for precise alignment of multiple patterned papers, which would be necessary to achieve multi-target assays on a single device in the case of reversed layer order (patterned LUMABS layer on top of patterned furimazine layer). Following formation of the antibody-LUMABS complex, the device is flipped and the bioluminescent signal collected by a standard digital camera (**FIG. 1C**). More detailed information on single device layers and on device fabrication in general is provided in **FIGs. 5A-C.**

The 3D-μPAD design has several important features:

   (i) short flow paths compared to lateral flow designs, which allows for rapid assays with low sample volumes, including viscous blood serum or plasma,
   (ii) on-device blood cell removal from whole blood samples,
   (iii) separate storage of luciferase-integrating BRET sensors and corresponding substrates (luciferin) in close proximity, and
   (iv) patterned signaling layer for simultaneous detection of multiple targets and the possibility to include positive and negative standards

Another challenge associated with μPADs in general is the fact that quantitative results depend on the applied sample volume, since signal intensity relies on the absolute amount of target analyte in the detection area rather than the concentration. We show that the closed space created by full lamination of a paper-based analytical device contributes to limiting the sample volume that can be absorbed by the paper substrate, which is important because it allows for sample volume independent measurements. The 3D-μPADs were therefore designed as closed compartments by complete device lamination for sample volume independent measurements.

**Results and Tests**

Before assembly of the integrated device, we first used simple spot tests with an HIV1-p17 antibody targeting LUMABS on wax-patterned paper to confirm that the antibody concentration-dependent response of LUMABS on the paper platform is comparable to the one observed in bulk solution. Having established that performance of LUMABS on paper is similar to that in solution, we next tested the performance in an integrated device. First, devices with the three signal detection areas modified with identical amounts of a single type of LUMABS (anti-HIV-LUMABS, anti-DEN-LUMABS or anti-HA-LUMABS, respectively) (**FIG. 5C (d-1)**) were evaluated, allowing the collection of triplicate data points from a single sample application (20 μL; **FIG.** 2A). The mean of the relative standard deviations for the hue values collected from the three detection areas of a single device was below 1% for all assays, indicating a low variation in sample distribution to the three detection areas (**Table 1**).

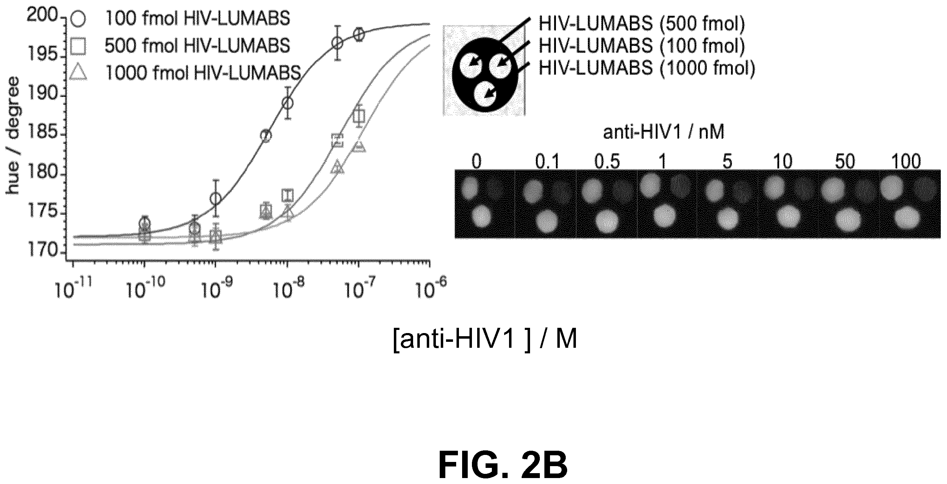

**Table 1.** Relevant analytical parameters for response curves shown in **FIGs. 2A** and **FIG. 2B.** [a] Absolute amount of LUMABS deposited on a single signal detection area. [b] Standard deviations for measurements performed in triplicate. [c] Mean relative standard deviation of hue values obtained by triplicate measurements over the entire response curve.

| LUMABS type (amount)[a] | $c_{50}$ / nM | hue$_0$ / degrees[b] | Mean relative st. dev. / % [c] |
|---|---|---|---|
| HIV (100 fmol) (Fig. 2a) | 6.35 | 172.8±0.8 | 0.63 |
| DEN (100 fmol) (Fig. 2a) | 35.57 | 175.4±0.9 | 0.48 |
| HA (250 fmol) (Fig. 2a) | 8.48 | 171.5±0.6 | 0.47 |
| HIV (100 fmol) (Fig. 2b) | 5.65 | 172.1±2.1 | 0.86 |

6

(continued)

| LUMABS type (amount)[a] | $c_{50}$ / nM | $hue_0$ / degrees[b] | Mean relative st. dev. / % [c] |
|---|---|---|---|
| HIV (500 fmol) (Fig. 2b) | 55.66 | 171.1±0.3 | 0.58 |
| HIV (1000 fmol) (Fig. 2b) | 116.16 | 172.0±1.3 | 0.48 |

Also, satisfactory batch-to-batch fabrication reproducibility was achieved for these manually assembled 3D-μPADs, showing a relative standard deviation of the mean of the c50 values below 1% ($c50 = 6.40 \pm 0.06$ nM) for three batches of anti-HIV1 detecting devices fabricated on three different days. Next, the colorimetric response of devices having three signal detection areas modified with different amounts of anti-HIV-LUMABS (100, 500 and 1000 fmol) (**FIG. 5C (d-2)**) was evaluated by titration with 0.1 - 100 nM of anti-HIV1p17 antibody. The effective response range was found to depend on the amount of sensor protein, with the high amounts of LUMABS requiring a higher concentration of antibody to go from the blue-green 'closed state' of the sensor to the blue light emitting antibody-bound 'open' state. This property could be exploited to enable measurements over an extended concentration range that cannot be achieved using a single signal detection area. The corresponding images in **FIG. 2B** show the actually observed bioluminescence emission signals providing the basis for the measurement of hue values.

The availability of three detection areas can also be used to allow the multiplex detection of 3 different antibodies. **FIG. 2C** shows an experiment in which the signal detection areas of the lower paper layer were modified with three previously developed LUMABS sensors targeting anti-HIVlp17, anti-HA and anti- Dengue antibodies (**FIG. 5C (d-3)**). While the extent of the sensor response to their target antibody varies for the different LUMABS sensors, the selectivity is excellent, showing no cross- talk between different detection areas (**FIG. 2C**).

An important advantage of the ratiometric, hue-based readout approach used here is that the signal should be independent of the bioluminescence intensity. This is particularly important for bioluminescent sensors, as bioluminescence intensity typically decreases in time as a result of substrate turn- over and/or product inhibition. The results shown in the images of **FIG. 2B** for 0 nM anti-HIV1 illustrate this point. Whereas the signal intensity varies substantially between the three detection areas because of the different amounts of sensor protein, the color of the signal as represented by the hue value is the same and independent of signal intensity (**Table 1**). The hue values are stable and can be reliably detected as long as sufficient light is emitted, which is because the bright and glow-type bioluminescence of the NanoLuc/furimazine system extends beyond 30 minutes.

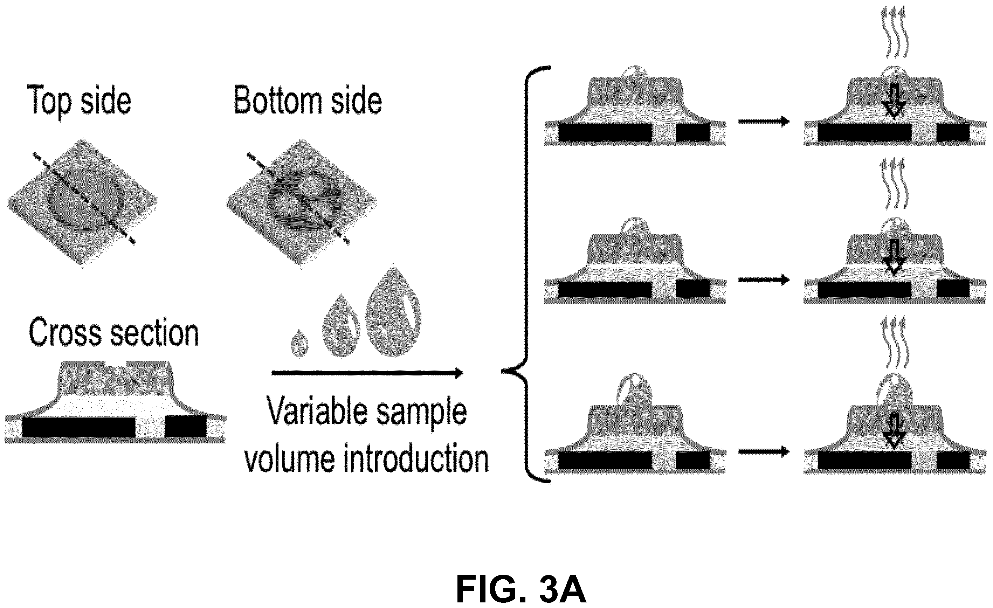

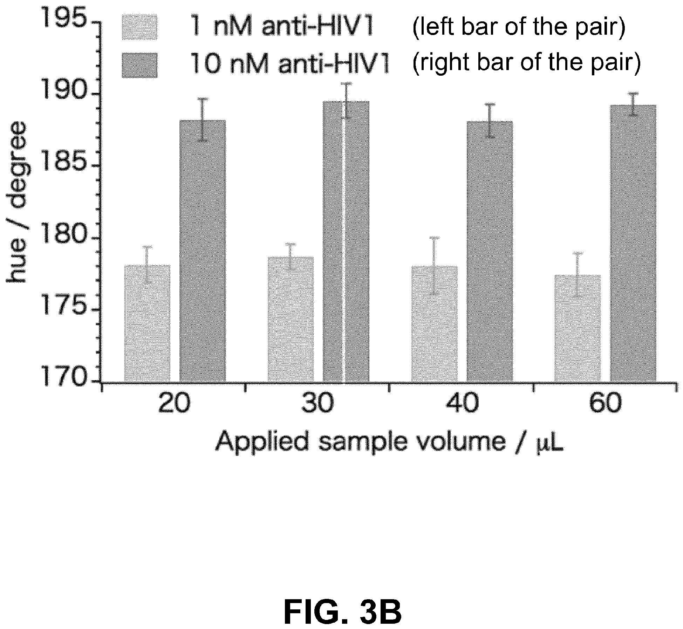

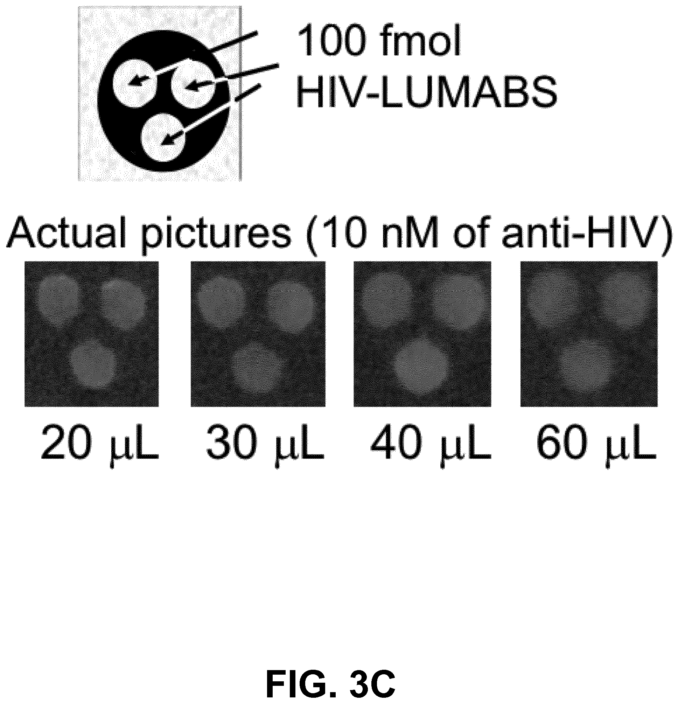

Volume-independent analysis and long-term stability represent two important aspects of device performance for point- of-care applications. The results shown in **FIGs. 3A-C** demonstrate that our 3D-μPADs allow sample volume-independent analyses, provided the applied volume is above the threshold value required for full wetting of all paper layers and the transport of target antibody and furimazine substrate to the LUMABS-modified detection areas. In the μPAD system, no significant differences in colorimetric response were found when varying the volume of applied sample liquid up to a factor of three (20 μL-60 μL). In a study on the storage stability of 3D-μPADs, fully assembled devices modified with anti-HIV-LUMABS were kept under inert gas (Ar) atmosphere at -20 degrees Celsius for a period of 2 months and the response to anti-HIV1 antibodies was tested sporadically. Our data reveals that no significant deterioration was observed.

To evaluate the suitability of the 3D-μPADs for antibody detection in biological samples, we next performed experiments with antibody-spiked porcine serum samples. The absolute bioluminescence emission intensities decreased when switching from aqueous buffer solutions to the blood serum matrix, which has been attributed to a lower activity of the NanoLuc enzyme in that matrix. For this reason, the amount of LUMABS deposited onto the signal detection areas was increased to 500 fmol for all three of the investigated sensor variants. Linear regression fits were applied to the linear segments of the experimental data. Response curves expanded for the low antibody concentration range were used to determine limits of detection using the 3s method, yielding LODs of 2.8 nM, 7.1 nM and 19.3 nM for the detection of anti-HIV1, anti-HA and anti-DEN1, respectively. Having established the performance in blood serum, we finally tested the performance of the 3D-μPADs using whole blood samples spiked with three different antibodies at variable concentrations. Here, red and white blood cells, as well as platelets are removed by the blood separation membrane integrated into the paper device. First, we established the minimally required volume of whole blood by applying different volumes of whole blood spiked with 50 nM of anti-HA (**FIG. 4A**). Not surprisingly, considering the solid constituents of whole blood (haematocrit value), the application of 20 μL of sample did not result in sufficient sample transport to signal detection areas. Reliable assays required the application of 30 μL or higher volumes of whole blood as shown by the constant hue signal obtained with 30 μL to 60 μL of whole blood. In addition, incubation times were increased from 15 min to 21 min to cope with the higher viscosity of whole blood compared to aqueous buffer solutions or serum and the time required for blood separation within the device. **FIG. 4B** shows that hue values increased during the first 18 min after whole blood sample application, while the response was nearly instantaneous in the case of aqueous antibody solutions.

**FIG. 4C** shows the results of quantitative recovery experiments in which whole blood samples were spiked with different concentrations of three different antibodies. Quantification was done using calibration data obtained with porcine serum samples, assuming that there are no significant differences in response behavior towards blood serum and blood plasma generated by the plasma separation membrane, respectively. Although the standard deviation is larger for the whole blood samples, there is a reasonable agreement between the spiked and the actually measured mean antibody concentration values for both blood samples containing single target antibodies and mixtures of multiple antibodies (**FIG. 4C**).

Described herein is a fully integrated paper-based analytical system relying on ratiometric bioluminescence detection in a single drop of whole blood in a highly user-friendly "just add the sample" manner. Because the response of the system is independent of the applied sample volume over a wide volume range, no complex reagent handling and no quantitative sample metering are required. Although a standard digital camera was used in this study for signal acquisition, other embodiments or applications of the 3D-μPAD devices may use a smartphone camera, allowing integration of signal detection and application-based analysis without a dedicated measurement instrument. Applications of these 3D-μPADs are also not restricted to LUMABS-based detection of antibodies but may be combined with any BRET-based sensor protein, including recently developed sensors for small-molecule drugs (LUCIDs) and nucleic acids (BRET-beacons).

## Experimental Procedures

Devices not containing a plastic film layer with sewn-in cotton thread as claimed are for comparison reasons only.

### *Materials and instruments*

Ultrapure water (18.2 MΩ cm) from a PURELAB flex water purification system was used throughout all experiments. LUMinescent AntiBody Sensing proteins (LUMABS) against HIV1-p17 antibody (anti-HIV-LUMABS), dengue virus type 1 antibody (anti-DEN-LUMABS) and hemagglutinin (anti-HA-LUMABS) were prepared following the previously reported procedure[1] and stored in freeze dried form at -80 degrees Celsius. NanoGlo Luciferase assay substrate (N1110, Furimazine, with enclosed Promega buffer) was purchased from Promega Corporation (Tokyo, Japan). anti-Dengue Virus Type I (Antibody, clone 15F3-1) (anti-DEN1) was purchased from Merck Millipore (Tokyo, Japan). Hemagglutinin (HA) Tag Monoclonal Antibody (2-2.2.14) (anti-HA) was purchased from Thermo Fisher Scientific (Kanagawa, Japan). HIV1-p17 (clone 32/1.24.89) antibody (anti-HIV1) was purchased from Zeptometrix (New York, America). PBS ×10 (phosphate buffered saline, pH 7.4) was purchased from Nippon Gene (Tokyo, Japan). Porcine whole blood with 0.3 wt % citric acid and porcine serum were purchased from Tokyo Shibaura Zouki Co (Tokyo, Japan). All other reagents were purchased from Wako Pure Chemical Industries Ltd. (Osaka, Japan).

The filter paper used was a Whatman No.1 filter paper (GE Healthcare, Tokyo, Japan). Hydrophobic wax patterns on A4 sized filter papers were designed in Adobe Illustrator CC software and printed with a ColorQube 8570 wax printer (Xerox, Norwalk, CT, USA), followed by a heating process where printed wax was melted into the paper by placing it on a hot plate (Nissin 99 NHS-450ND) at 150 degrees Celsius for 180 s. Vivid plasma separation membranes grade GR (Pall Corporation, Tokyo, Japan) were cut into 7.9 mm circles by a hand craft punch (McGill, d = 7.9 mm). To encase devices comprising multiple layers, hot lamination with a QHE325 laminator (Meiko Shokai, Tokyo, Japan) using 100 μm thick (including top and bottom layer) hot lamination sheets (Jointex, Tokyo, Japan) was performed. Inlet holes on the top lamination sheet layer were cut by a Silhouette Cameo electronic knife blade cutting device (Silhouette, Lehi, UT, USA). For capturing bioluminescence signals from multi-layer 3D-μPADs, a DMC-FZ50 digital camera (Panasonic, Osaka, Japan) was used.

### *Paper layer modification*

In general, to prevent the non-specific adsorption of target antibodies and LUMABS, all filter papers (wax-patterned or non-patterned) were first soaked for 15 min in 0.5 wt% bovine serum albumin (BSA) solution in PBS for blocking, subsequently immersed for 15 min into water to wash out excess amounts of BSA and allowed to completely dry for 30 min at 40 degrees Celsius, before any further modification. The filter paper was wax-patterned into spots of 4.0 mm diameter. LUMABS was applied as solution in PBS buffer containing 1 mg/mL of BSA. **FIG. 5A** shows the modification of the upper paper layer for 3D-μPADs carrying the light emitting NanoLuc substrate furimazine. BSA-treated paper was cut into round shapes of 7.9 mm diameter, 15 μL of 50 times diluted furimazine in ethanol was applied, followed by complete drying. **FIG. 5B** shows the schematic of the second paper layer (lowermost paper layer of the 3D-μPAD) patterned into multiple signal readout areas (**FIG. 5C**). In cases of running triplicate assays for a single target antibody from a single sample application, all of the signal readout areas were impregnated with identical LUMABS solutions (in PBS buffer with 1mg/ml BSA) (**FIG. 5C (d-1)**). When running assays for a single target antibody with different amounts of sensing proteins, different volumes of identical LUMABS solutions were deposited in different signal readout areas (**FIG. 5C (d-2)**). Finally, for the simultaneous detection of multiple targets in parallel, multiple LUMABS targeting different antibodies (anti-HIV, anti-DEN and anti-HA) were deposited in different signal readout areas (**FIG. 5C (d-3)**).

### Multi-layer device assembly

Devices were assembled in sets of 8 pieces. Onto a wax-patterned and LUMABS impregnated paper sheet (**FIG. 5B**) with two rows of 4 wax patterns, furimazine impregnated paper discs (**FIG. 5A**) were set to be centered on each of the underlying wax patterns, followed by placing a plasma separation membrane disc on top of each paper disc. This 3-layered structure (LUMABS impregnated paper, furimazine impregnated paper, blood separation membrane) was sandwiched between the two layers of the hot lamination film with the cut-out sample inlet holes of the top layer being aligned to the plasma separation membrane discs and passed through the hot laminator.

### Optical signal acquisition

After passing of the respective incubation time, the bioluminescence emission signal was monitored in 3-minute intervals by taking a picture in a darkroom box made out of Styrofoam for eliminating ambient light interference. The settings of the digital camera were as follows: white balance fixed; exposure time 60 s; ISO 1600; f-value 2.8. Images were captured in camera RAW format, followed by conversion into JPEG format by the image processor of the Adobe Photoshop CC software (color temperature 3200 K, tint -150). Using the converted JPEG file, the hue value (on the 0-360 degrees scale) of the signal readout area(s) was measured with the image processing software ImageJ (National Institutes of Health).

### Spot tests and microplate assay

For initial paper spot tests, 1 $\mu$L of 100 nM of anti-HIV-LUMABS (corresponding to an absolute amount of 100 fmol of sensor protein) was deposited into the test spots. After drying completely, 0.3 $\mu$L furimazine (30 times diluted by Promega buffer), followed by 4 $\mu$L of sample containing HIV1-p17 antibody at different concentrations (in PBS with 1 mg/mL BSA) were added to the test spots. For comparison purposes, the assay was also performed in a white 96-well microplate. 20 $\mu$L of HIV1-p17 antibody sample solutions were supplemented with furimazine and LUMABS at a final concentration of 25 nM. In all cases (paper platform and microplate), photographs were taken in the darkroom box starting 15 min after deposition of all reagents.

### Assays with multi-layer devices in aqueous buffer solutions or in porcine serum

If not mentioned otherwise, 20 $\mu$L of the corresponding sample solution (in PBS buffer with 1 mg/mL BSA or in porcine serum) with a specified antibody concentration was applied to the sample inlet area of device. Photographs were taken in the darkroom box starting 15 min after sample application. For experiments evaluating the influence of applied sample amounts, sample volumes were varied between 20 $\mu$L (minimum) and 60 $\mu$L (maximum).

### Selectivity and cross-reactivity evaluation

The response of 3D-$\mu$PADs with signal detection areas modified with different LUMABS (**FIG. 5C (d-3)**) to aqueous solutions of a single antibody or to mixed antibody solutions was evaluated. To adjust for differences in absolute hue values between different sensor proteins, $\Delta$hue values normalized to signals obtained with antibody-free solutions have been used.

### Storage stability evaluation

Fully assembled 3D-$\mu$PADs modified with anti-HIV-LUMABS were placed into capped vials filled with argon gas containing silica gel desiccant and stored in a freezer at -20°C. Bioluminescence signal evolvement in aqueous solutions was evaluated at room temperature after various storage periods. Concentrations of pre-deposited furimazine and anti-HIV-LUMABS were as mentioned above for multi-layer devices.

### Antibody-spiked porcine whole blood assays with multi-layer devices

Porcine whole blood containing 0.3 wt % citric acid as an anticoagulant was spiked with various amounts of either a single antibody or a mixture of antibodies. Experiments were performed as in the case of working with aqueous buffers or serum, however with the applied sample volume generally being 30 $\mu$L (20-60 $\mu$L for experiments evaluating the sample volume dependency) and the incubation time before starting photograph acquisition being 21 min to account for the volume reduction by blood cell separation and lower sample flow speeds due to increased viscosity, respectively.

*Curve fitting procedure*

Curve fitting of the experimentally obtained data (antibody concentration vs. hue values) was done using **equation 1** shown below, where $c_{50}$ indicates the antibody concentration resulting in 50% of the maximal hue change and $\Delta hue_{max}$ and $hue_0$ represent the maximal change in the measured hue value and the hue value in the absence of target antibody, respectively.

$$hue = \frac{\Delta hue_{max}[antibody]}{c_{50} + [antibody]} + hue_0 \qquad (equation\ 1)$$

Other aspects are provided in US Provisional Application 62/550,001 filed August 25, 2017.

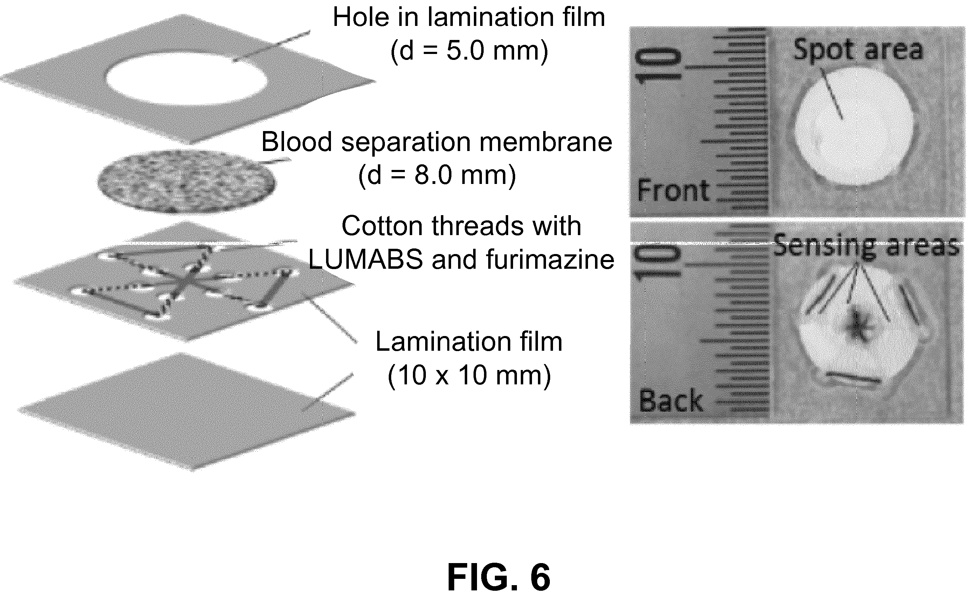

The detection device of the invention has a bioluminescent protein integrating microfluidic thread-based analytical device ($\mu$TAD) for point-of-care testing (POCT) as shown in **FIG. 6**. In the exemplary embodiment of **FIG. 6** for a blood sample, this device has a blood separation membrane and a plastic film layer with sewn-in cotton thread, in which proteins and luciferin for signal generation are pre-deposited. The innate strength and flexibility of sewn thread patterns enable the design of miniaturized devices with minimal sample volume requirement. Use of threads with hydrophilic/hydrophobic segments patterned by a permanent ink marker resulted in homogeneous LUMABS distribution allowing simultaneous assaying of multiple antibodies using different LUMABS.

The LUMABS-based $\mu$TADs developed in this embodiment offer user-friendly, quantitative and simultaneous POCT for multiple antibodies from a single drop of whole blood small enough to be collected from a finger prick. This $\mu$TAD has four vertically arranged layers including lamination films for device fixation, a blood separation membrane for separating plasma from whole blood, and a sewn cotton thread layer in which LUMABS and furimazine (substrate for the luciferase) are pre-deposited in dry form, spatially separated in two intertwisted threads to prevent their premature mixing (**FIG. 6**).

*Device fabrication*

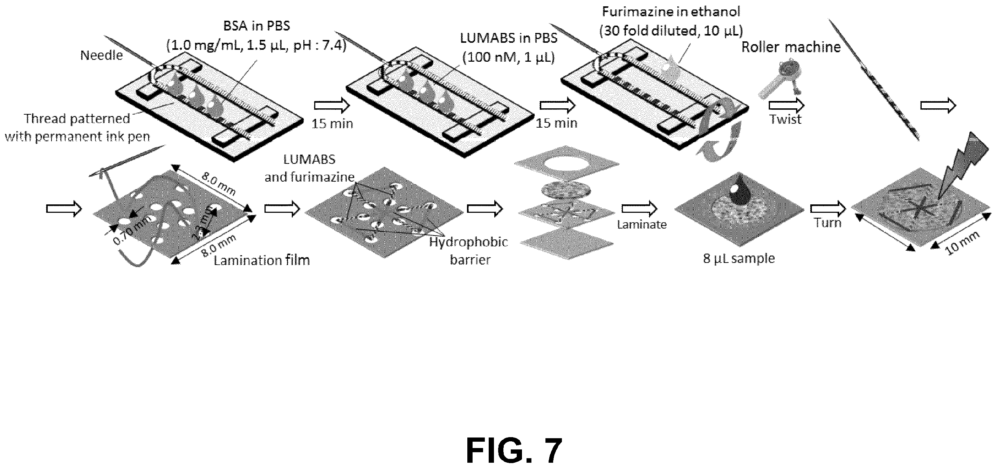

A schematic of the $\mu$TAD fabrication is shown in **FIG. 7**. At first, 1.5 $\mu$L of BSA in PBS (1 mg/mL, pH 7.4) is applied to each of the six hydrophilic areas on commercially available cotton thread patterned with hydrophobic barriers using a permanent ink marker. After drying for 15 min at room temperature (RT), 1 $\mu$L of the respective LUMABS (HIV-LUMABS, HA-LUMABS, or DEN-LUMABS; 100 nM solution in PBS of pH 7.4 containing 1 mg/mL BSA) is introduced to each of the blocked hydrophilic sections. Subsequently, 10 $\mu$L furimazine in ethanol (30 fold diluted with ethanol) is deposited on the other half of the thread. After 15 min drying at RT, the two threads are twisted 50 times by the roller machine to bring LUMABS and furimazine in close proximity. Then, the twisted thread is sewn into the lamination film with pre-punched holes. Finally, the four layers including lamination films, blood separation membrane and cotton thread layer are assembled and heat laminated, to obtain the $\mu$TAD.

*Sample preparation*

Antibodies against HIV1-p17 (HIV), hemagglutinin (HA), and dengue virus type I (DEN) were spiked at different concentrations into porcine whole blood, except for assaying multiple antibodies with different LUMABS, where measurements were performed in PBS (pH 7.4) containing 1 mg/mL of BSA.

*Bioluminescence detection*

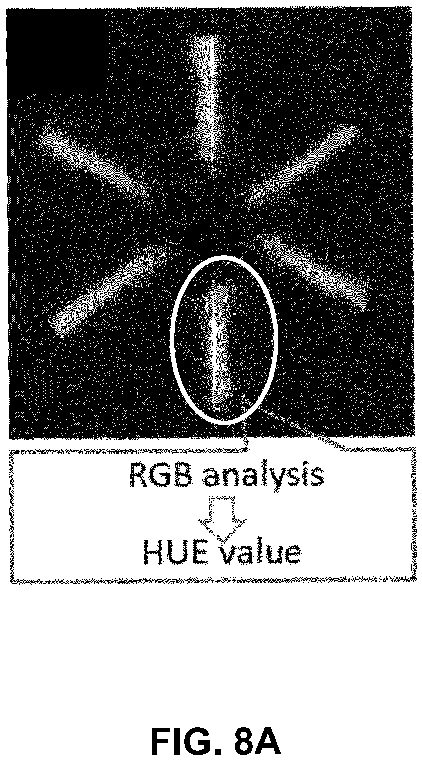

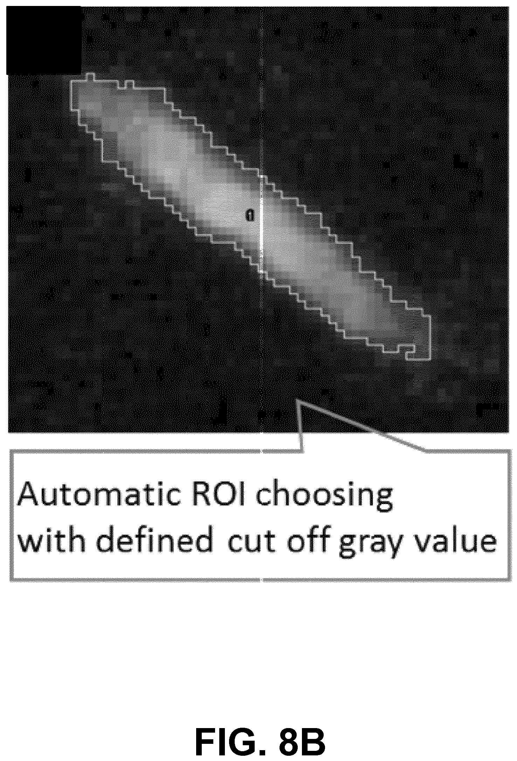

Bioluminescence emission was recorded by a digital camera (60 s exposure) in the dark. The presence of target antibodies is indicated by a change in the color of the emitted bioluminescence from green to blue, which is quantified by means of the HUE parameter extracted from the acquired RGB values (**FIG. 8A**). The six regions of interest (ROI) on a single device are detected automatically by the ImageJ software through a defined cut off gray value (**FIG. 8B**). The error bars in response curves represent standard deviations from six signal detection areas of a single device, except for the detection of multiple different antibodies, where standard deviations were obtained from two sensing areas of three devices.

**Results**

To evaluate the bioluminescence response on $\mu$TADs, 8 $\mu$L whole blood samples spiked with antibodies at concentrations of 1 to 500 nM for anti-HA and anti-HIV or 5 to 1000 nM for anti-DEN were applied onto the sampling area.

Based on linear curve fits applied to the low sample concentration ranges of the response curves, detection limits of 10 nM, 6.1 nM and 50 nM, respectively, were obtained for the three antibodies in whole blood. The antibody concentration-dependent change of the bioluminescence emission color from green to blue confirms the mixing and reaction of the spatially separately pre-deposited LUMABS and furimazine in the intertwisted threads upon target antibody introduction from whole blood samples, as well as the reliable removal of red blood cells by the blood separation filter integrated into the μTADs, which would otherwise absorb the emitted bioluminescence. Furthermore, the obtained homogeneous signals from the six signal detection areas show that mixing of LUMABS deposited on each of the six hydrophilic areas separated by hydrophobic barriers patterned by the permanent ink marker is prevented and that target antibodies from whole blood samples are equally distributed to each of the signal detection areas.

The batch-to-batch reproducibility of the manually assembled devices was evaluated using whole blood samples at concentrations of 1 to 500 nM for anti-HIV. The investigated three batches of devices were independently fabricated on different days and tested on different days. The absence of statistically significant variations of the hue values observed for three replicate measurements are an indication for the excellent manufacturing reproducibility of the devices. The HUE value-based colorimetric bioluminescence emission signals in whole blood samples at concentrations of 0, 25 and 500 nM of anti-HIV were found to be stable between 6-18 min after sample application, indicating that there is no need for exactly timed signal readout.

Finally, the performance of μTADs for the simultaneous detection of three different antibodies (all at 500 nM in PBS) was evaluated to confirm the absence of cross-talk between signal detection areas targeting different analytes. For this purpose, two of six detection areas on one device are modified with identical LUMABS. This result indicates that no cross-talk is observed in the case of assaying multiple different antibodies (3x2) on single μTADs.

**Claims**

1. A detection device for bioluminescent detection of analytical targets from a fluid sample using luminescent sensing proteins, comprising:

   (a) a first lamination layer with an opening for receiving the fluid sample;
   (b) a separation membrane for separating part of the fluid sample received through the opening of the first lamination layer;
   (c) a plastic film layer with sewn-in cotton thread, wherein the thread comprises two intertwisted threads, one impregnated with luminescent sensing proteins and the other impregnated with luciferin, thereby spatially separating the luminescent sensing proteins and luciferin to prevent their premature mixing, wherein, upon receiving the fluid sample, the luciferin and luminescent sensing proteins generate a bioluminescent signal for the detection of the analytical targets; and
   (d) a second lamination layer, which together with the first lamination layer encases and immobilizes the separation layer, and the plastic film layer with sewn-in cotton thread into a single vertically stacked assembly detection device.

2. The detection device as set forth in claim 1, wherein the thread is sown into pre-punched holes of the plastic film layer.

3. The detection device as set forth in any one of claims 1 to 2, wherein the fluid sample is a biological fluid sample or a non-biological fluid sample.

4. The detection device as set forth in any one of claims 1 to 3, wherein the separation membrane is a blood cell filtration membrane.

5. The detection device as set forth in any one of claims 1 to 4, wherein the single vertically stacked assembly detection device enables a pump-free transport mechanism of the fluid sample by means of capillary forces, gravity or a combination thereof.

6. The detection device as set forth in any one of claims 1 to 5, wherein the analytical targets are biomarkers, antibodies, antigens, proteins, drugs or nucleic acids.

7. The detection device as set forth in any one of claims 1 to 6, wherein the detection device comprises a reference scale or reference system for the bioluminescent emission signals.

**Patentansprüche**

1. Nachweisvorrichtung zum biolumineszenten Nachweis analytischer Targets aus einer Fluidprobe unter Verwendung lumineszierender Sensorproteine, umfassend:

   (a) eine erste Laminierungsschicht mit einer Öffnung zum Aufnehmen der Fluidprobe;
   (b) eine Trennmembran zum Trennen eines Teils der Fluidprobe, die durch die Öffnung der ersten Laminierungsschicht aufgenommen wird;
   (c) eine Kunststofffolienschicht mit eingenähtem Baumwollfaden, wobei der Faden zwei verdrillte Fäden umfasst, von denen einer mit lumineszierenden Sensorproteinen imprägniert ist und der andere mit Luciferin imprägniert ist, wodurch die lumineszierenden Sensorproteine und Luciferin räumlich getrennt werden, um ihre vorzeitige Vermischung zu verhindern, wobei das Luciferin und die lumineszierenden Sensorproteine bei Empfang der flüssigen Probe ein biolumineszierendes Signal für den Nachweis der analytischen Targets erzeugen; und
   (d) eine zweite Laminierungsschicht, die zusammen mit der ersten Laminierungsschicht die Trennschicht und die Kunststofffolienschicht mit eingenähtem Baumwollfaden zu einer einzigen vertikal gestapelten Anordnungs-Nachweisvorrichtung umhüllt und immobilisiert.

2. Nachweisvorrichtung nach Anspruch 1, wobei der Faden in vorgestanzte Löcher der Kunststofffolienschicht eingenäht wird.

3. Nachweisvorrichtung nach einem der Ansprüche 1 bis 2, wobei die Fluidprobe eine biologische Fluidprobe oder eine nichtbiologische Fluidprobe ist.

4. Nachweisvorrichtung nach einem der Ansprüche 1 bis 3, wobei die Trennmembran eine Blutzellfiltrationsmembran ist.

5. Nachweisvorrichtung nach einem der Ansprüche 1 bis 4, wobei die einzelne vertikal gestapelte Anordnungs-Nachweisvorrichtung einen pumpenfreien Transportmechanismus der Fluidprobe mittels Kapillarkräften, Schwerkraft oder einer Kombination davon ermöglicht.

6. Nachweisvorrichtung nach einem der Ansprüche 1 bis 5, wobei die analytischen Targets Biomarker, Antikörper, Antigene, Proteine, Arzneimittel oder Nukleinsäuren sind.

7. Nachweisvorrichtung nach einem der Ansprüche 1 bis 6, wobei die Nachweisvorrichtung eine Referenzskala oder ein Referenzsystem für die biolumineszenten Emissionssignale umfasst.

**Revendications**

1. Dispositif de détection pour la détection bioluminescente de cibles analytiques à partir d'un échantillon de fluide à l'aide de protéines de détection luminescentes, comprenant :

   (a) une première couche de laminage avec une ouverture permettant de recevoir l'échantillon de fluide ;
   (b) une membrane de séparation pour séparer une partie de l'échantillon de fluide reçu à travers l'ouverture de la première couche de laminage ;
   (c) une couche de film plastique avec un fil de coton cousu, dans lequel le fil comprend deux fils entrelacés, l'un imprégné de protéines de détection luminescentes et l'autre imprégné de luciférine, séparant ainsi spatialement les protéines de détection luminescentes et la luciférine afin d'empêcher leur mélange prématuré, dans lequel, lors de la réception de l'échantillon de fluide, la luciférine et les protéines de détection luminescentes génèrent un signal bioluminescent pour la détection des cibles analytiques ; et
   (d) une seconde couche de laminage, qui, conjointement avec la première couche de laminage, enferme et immobilise la couche de séparation et la couche de film plastique avec le fil de coton cousu dans un unique dispositif de détection à assemblage empilé verticalement.

2. Dispositif de détection tel que défini dans la revendication 1, dans lequel le fil est cousu dans des trous pré-perforés de la couche de film plastique.

3. Dispositif de détection tel que défini dans l'une quelconque des revendications 1 à 2, dans lequel

l'échantillon de fluide est un échantillon de fluide biologique ou un échantillon de fluide non biologique.

4. Dispositif de détection tel que défini dans l'une quelconque des revendications 1 à 3, dans lequel la membrane de séparation est une membrane de filtration de cellules sanguines.

5. Dispositif de détection tel que défini dans l'une quelconque des revendications 1 à 4, dans lequel l'unique dispositif de détection à assemblage empilé verticalement permet un mécanisme de transport sans pompe de l'échantillon de fluide au moyen de forces capillaires, de la gravité, ou d'une combinaison de celles-ci.

6. Dispositif de détection tel que défini dans l'une quelconque des revendications 1 à 5, dans lequel les cibles analytiques sont des biomarqueurs, des anticorps, des antigènes, des protéines, des médicaments ou des acides nucléiques.

7. Dispositif de détection tel que défini dans l'une quelconque des revendications 1 à 6, dans lequel le dispositif de détection comprend une échelle de référence ou un système de référence pour les signaux d'émission bioluminescents.

FIG. 1A

Hole in lamination film
($d$ = 5.5mm)

Thickness: 50 μm

Plasma separation membrane
($d$ = 7.9 mm) — 330 μm

Upper paper layer impregnated with furimazine ($d$ = 7.9 mm) — 180 μm

Lower paper layer patterned for antibody detection impregnated with LUMABS

Wax in paper

Lamination film — 180 μm

Furimazine

Lamination to encase and immobilize all layers

**FIG. 1B**

EP 3 673 077 B1

**Applying whole blood**     **Incubation**     **Flip over**     **Analysis**

# FIG. 1C

EP 3 673 077 B1

FIG. 2A

EP 3 673 077 B1

FIG. 2B

FIG. 2C

FIG. 3A

**FIG. 3B**

100 fmol HIV-LUMABS

Actual pictures (10 nM of anti-HIV)

20 µL    30 µL    40 µL    60 µL

FIG. 3C

FIG. 4A

FIG. 4B

| Sample No. | 1 | | 2 | | 3 | | 4 | | 5 | | 6 | | 7 | | 8 | |
|---|---|---|---|---|---|---|---|---|---|---|---|---|---|---|---|---|
| | spiked | found | spiked | found | spiked | found | spiked | found | spiked | found | spiked | found | spiked | found | spiked | found |
| anti-HIV1/nM | 0 | nd | 150 | 144±3 | 100 | 99±15 | 0 | nd | 0 | 1±0 | 0 | 1±0 | 100 | 95±20 | 50 | 52±28 |
| anti-HA/nM | 0 | nd | 0 | 1±1 | 0 | 1±0 | 150 | 140±49 | 0 | 1±1 | 50 | 43±26 | 0 | 1±1 | 50 | 58±13 |
| anti-DEN1/nM | 0 | 4±3 | 0 | 7±5 | 0 | 13±1 | 0 | 3±1 | 150 | 159±58 | 100 | 81±47 | 50 | 50±19 | 50 | 43±13 |

## FIG. 4C

EP 3 673 077 B1

**FIG. 5A**

BSA (0.5 wt%)
15 min

Water
15 min

40 °C
30 min

Punch
(diameter = 7.9 mm)

Furimazine
50 times diluted
by ethanol

**FIG. 5B**

Wax-patterned paper

BSA (0.5 wt%)
15 min

Water
15 min

40 °C
30 min

LUMABS

**FIG. 5C**

4 mm

10 mm

(d-1)    (d-2)    (d-3)

EP 3 673 077 B1

Hole in lamination film
(d = 5.0 mm)

Blood separation membrane
(d = 8.0 mm)

Cotton threads with
LUMABS and furimazine

Lamination film
(10 x 10 mm)

Spot area

Front

Sensing areas

Back

**FIG. 6**

EP 3 673 077 B1

FIG. 7

FIG. 8A

Automatic ROI choosing
with defined cut off gray value

# FIG. 8B

**EP 3 673 077 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015007317 A

- US 62550001

**Non-patent literature cited in the description**

- *Nat. Chem. Biol.*, 2014, vol. 10, 598-603